# EUROPEAN PATENT SPECIFICATION

(11) **EP 0 455 830 B1**
(45) Date of publication and mention of the grant of the patent: **26.06.1996**
(21) Application number: 90917683.6
(22) Date of filing: 30.11.1990
(51) Int. Cl.: A61K 31/54, C07D 417/12, C07D 513/04

(54) **MEDICINE CONTAINING THIAZINE DIOXIDE DERIVATIVE**
EIN THIAZINDIOXIDDERIVAT ENTHALTENDES MEDIKAMENT
MEDICAMENT CONTENANT UN DERIVE DE DIOXYDE DE THIAZINE

(30) Priority: 30.11.1989 JP 311883/89; 30.11.1989 JP 311884/89
(43) Date of publication of application: 13.11.1991
(73) Proprietor: NIPPON HYPOX LABORATORIES INCORPORATED, Tokyo 192-03 (JP)
(72) Inventor: SATOH, Toshio 57-3, Nagao Jorokucho, Tokushima-ken 771-42 (JP); MATSUMOTO, Hitoshi 125-22, Shimojukuman, Tokushima-shi Tokushima-ken 770 (JP); KAKEGAWA, Hisao 404 Dia Palace Nakamaekawa, Tokushima-shi Tokushima-ken 770 (JP); NIIRO, Yasunori 4-33-206, Minamisuehiro-cho, Tokushima-ken 770 (JP)
(74) Representative: Türk, Dietmar, Dr. rer. nat.
(86) International application number: JP9001562
(87) International publication number: WO9107970

(56) References cited:
- EP-A- 0 294 994
- WO-A-90/09794
- BE-A- 0 832 707
- DE-A- 3 407 505
- JP-A- 1 228 975
- PATENT ABSTRACTS OF JAPAN, vol. 15, no. 112 (C-815)(4640), 18th March 1991, page 152 C 815; & JP-A-3 5425 (NIPPON HAI POTSUKUSU K.K.) 11-01-1991
- JAPANESE J. PHARMACOLOGY, vol. 59, suppl. 1, 1992, page 419 P, abstract no. P 520; Y. SHIROSHITA et al.: "Effects of HX-1920 on cisplatin-induced toxicity in mice, rats and ferrets"

## Description

The present invention relates to the use of thiazine dioxide derivaties for preparing a medicament suitable for the treatment of renal diseases and disorders of digestive organs.
(1) As is well known, the kidney of a living body has the function of excreting wastes from the blood and harmful matters taken into the living body such as chemicals or toxicants into urine by means of filtration and secretion mechanisms in the uriniferous tubule.
   However, the above wastes and chemicals are likely to cumulate locally. For this reason, renal diseases are often liable to occur. Specific examples of substances which cause such renal diseases include diverse substances. Chemicals as such are as follows.
   (Chemicals likely to cause renal dysfunctions)
   Analgesic, Antipyretic, Antiphlogistic and Antirheumatic drugs:
   phenacetin, aspirin, indomethacin, mefenamic acid, fenoprofen, gold preparation, D-penicillamine, etc.
   Antibiotics:
   aminoglycosides, polypeptides, polyenes, cephalosporins, penicillins, etc.
   Preparations for chemotherapy:
   sulfamides, etc.
   Carcinostatic or Anti-cancer drugs:
   mitomycin C, daunomycin, cisplatin, nitrosoureas, etc.
   Immunosuppressants:
   cyclophosphamide, etc.
   Narcotics:
   methoxyflurane, etc.
   Diuretics:
   thiazides, etc.
   Contrast medium
   The above chemicals which cause renal diseases are classified on the basis of the following Japanese literature.
   That is, the literature is "Rinsho Seijinbyo" Vol. 16, No. 8 (1986), "Rinsho to Kenkyu" Vol. 63, No. 4 (issued April 1986), and "Iyakuhin Yoran" (edited by Osaka-fu Hospital Pharmacist Society, Yakugyojihosha, issued November 10, 1983).
   Renal diseases have been discussed above from the viewpoint of the metabolic function of this organ. However, renal diseases include other diseases which are considered to be caused through an immunological mechanism.
   Renal diseases referred to in the present invention include diseases which exhibit dysfunctions in the glomeruli, uriniferous tubulus, loops, etc., such as (a) renal dysfunctions caused by the above metabolic function failure, e.g. acute nephritis caused by chemicals, etc., and chronic nephritis caused from a change of such acute nephritis into a chronic state, (b) acute nephritis caused through immunological mechanism and chronic nephritis caused from a change of such acute nephritis into a chronic state, and (c) acute nephritis caused by bacteria and virus infection, and chronic nephritis caused from a change of such acute nephritis into a chronic state, and the like.
   Meanwhile, as a method for the therapy of renal diseases, there are presently known methods for maintaining renal functions depending upon the severity of the disease, such as rest therapy, alimentary therapy, pharmacological therapy, hemodialysis, and kidney transplantation. The above hemodialysis therapy is considered to be a final treatment for renal diseases. In this therapy, however, wastes retained in the body due to a renal dysfunction are only eliminated from the blood, and the renal function is not improved. A patient would be required to have hemodialysis treatment for the rest of his or her life. Furthermore, in most cases, the patient would die finally from suffering from a complication such as cardiac failure, infection, etc. On the other hand, as pharmacological therapy, at present, there are employed treatments using diuretics, immunosuppressants, adrenocorticosteroids, etc., and treatments using mannitol, lactulose, etc. However, these are all symptomatic, and the effects of these treatments are low. A patient actually receives alimentary and rest therapy. For example, diuretics are under a trial use as a therapeutical preparation for renal diseases. However, it does not restore the decreased renal function.
   At present, therefore, it cannot be said that a medicament which is satisfactory as a therapeutical preparation against renal diseases is available, and it has been desired to develop a medicament having further efficacy.
   JP-A-1228975 describes a benzothiazine-1,1-dioxide derivative, its production and medical composition containing same.
   The compound 4-hydroxy-2-methyl-N-(2'-tetrazol-5'-yl-phenyl)-2H-1,2-benzothiazine-3-carboxamide -1,1-dioxide is described having an activity of inhibiting hyaluronidase. It is used as an anti-inflammatory or antiallergic agent.
   WO-A-9009794 describes a remedy for diabetes comprising a thiazine-1,1-dioxide derivative as active ingredient.
   It is a first object of the present invention to provide a therapeutical preparation for renal diseases, which exhibits excellent therapeutical effects on a wide range of renal diseases.
(2) It is known that the administration of a carcinostatic or other medicament causes vomiting, nausea, anorexia, stomach contraction, etc. And, antiemetics of various types have been conventionally developed and are clinically used. Of these, antiemetics of domperidone type have teratogenesis and disorder in endocrine function of diencephalon as a side effect. Metoclopramide, etc., are also drugs classiffied as a drastic drug, and these require various cares in clinical use. The effect of these antiemetics on inhibition of vomiting and nausea is very low.
   It is a second object of the present invention to provide a therapeutic preparation for a digestive organ, which can cure vomiting, nausea, anorexia, stomach contraction, etc., when a carcinostatic or other medicament is administered.

The present inventors have made a study to achieve the above first and second objects of the present invention, and found that the specific thiazine-1,1-dioxide derivative described below has a therapeutical effect on renal diseases and has a therapeutical effect on digestive organ inflammations. The present invention has been completed based on these findings.

That is, use of a thiazine-1,1-dioxide derivative of the general formula (I) 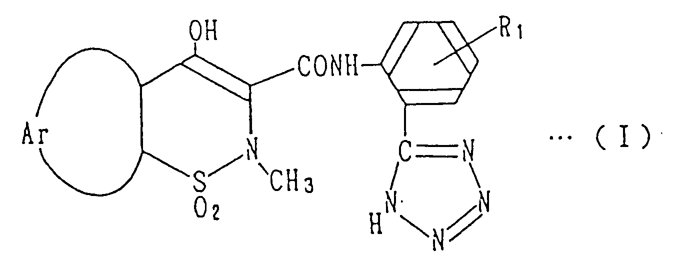
(wherein R₁ represents an atom or a substituent selected from the class consisting of a hydrogen atom, an alkyl group, an alkoxyl group, a halogen atom, a halogen-containing alkyl group, a hydroxyl group, a carboxyl group, alkylcarbonyl group and an ester group, Ar is (a) a benzene or naphthalene ring fused with the thiazine ring in the general formula (I) or (b) a hetero atom-containing aromatic ring selected from thiophene, furan, pyridine, quinoline, isoquinoline, and imidazole rings fused with the thiazine ring in the general formula (I)), or a pharmaceutically acceptable salt thereof for the manufacture of a medicament, for the treatment of renal diseases or for the treatment of disorders of digestive organs e.g. vomiting, nausea, anorexia, stomach contractions.

As R₁ in the above general formula (I), the alkyl group is a linear or branched alkyl group having 1 to 6 carbon atoms, the alkoxyl group is an alkoxyl group having a linear or branched alkyl group having 1 to 6 carbon atoms, the halogen atom is chlorine, fluorine, bromine or iodine, the halogen-containing alkyl group is a linear or branched alkyl group having 1 to 6 carbon atoms and being substituted with at least one halogen atom, the alkylcarbonyl group is an alkylcarbonyl group having a linear or branched alkyl group having 1 to 6 carbon atoms, and the ester group is an ester group of the formula of COOR₂ (in which R₂ is a linear or branched alkyl group having 1 to 6 carbon atoms).

Particularly preferred examples of the compound of the general formula (I) are as follows.
(1) 4-hydroxy-2-methyl-N-(2-(1H-tetrazol-5-yl)phenyl)2H-1,2-benzothiazine-3-carboxamide-1,1-dioxide.
   (R₁ = hydrogen atom, Ar = benzene ring)
(2) 4-hydroxy-2-methyl-N-(2-(1H-tetrazol-5-yl)-5-halogenophenyl)2H-1,2-benzothiazine-3-carboxamide-1,1-dioxide.
   (R₁ = 5-halogen atom, Ar = benzene ring)
(3) 4-hydroxy-2-methyl-N-(2-1H-tetrazol-5-yl)phenyl)-2H-1,2-pyrido-[2,3-e]-1,2-thiazine-3-carboxamide-1,1-dioxide.
   (R₁ = hydrogen atom, Ar = pyridine ring)
(4) - (35)
   4-hydroxy-2-methyl-N-(2-(1H-tetrazol-5-yl)phenyl-R₁-2H-pyrido-[2,3-e]-1,2-thiazine-3-carboxamide-1,1-dioxide.
   (R₁ = 2-halogen, 2-methoxy, 2-methyl,2-trifluoromethyl, 2-hydroxy, 2-carboxy, 2-ethoxycarbonyl, 2-acetyl, 3-halogen, 3-methoxy, 3-methyl, 3-trifluoromethyl, 3-hydroxy, 3-carboxy, 3-ethoxycarbonyl, 3-acetyl, 4-halogen, 4-methoxy, 4-methyl, 4-trifluoromethyl, 4-hydroxy, 4-carboxy, 4-ethoxycarbonyl, 4-acetyl, 5-halogen, 5-methoxy, 5-methyl, 5-trifluoromethyl, 5-hydroxy, 5-carboxy, 5-ethoxycarbonyl, or 5-acetyl, Ar = pyridine ring)
(36) 4-hydroxy-2-methyl-N-(2-(1H-tetrazol-5-yl)phenyl)2H-furo-[2,3-e]-1,2-thiazine-3-carboxamide-1,1-dioxide.
   (R₁ = hydrogen atom, Ar = furan ring)
(37) 4-hydroxy-2-methyl-N-(2-(1H-tetrazol-5-yl)phenyl)2H-5H-imidazo-[4,5-e]-1,2-thiazine-3-carboxamide-1,1-dioxide.
   (R₁ = hydrogen atom, Ar = imidazole ring)
(38) 4-hydroxy-2-methyl-N-(2-(1H-tetrazol-5-yl)phenyl)2H-thieno-[2,3-e]-1,2-thiazine-3-carboxamide-1,1-dioxide.
   (R₁ = hydrogen atom, Ar = thiophene ring)
(39) 4-hydroxy-2-methyl-N-(2-(1H-tetrazol-5-yl)phenyl)-6-trifluoromethyl-2H-thieno-[2,3-e]-1,2-thiazine-3-carboxamide-1,1-dioxide.
   (R₁ = hydrogen atom, Ar = trifluoromethyl group-substituted thiophene ring)
(40) 4-hydroxy-2-methyl-N-(2-(1H-tetrazol-5-yl)phenyl-6-chloro-2H-thieno-[2,3-e]-1,2-thiazine-3-carboxamide-1,1- dioxide.
   ((R₁ = hydrogen atom, Ar = chlorine-substituted thiophene ring)
(41) 4-hydroxy-2-methyl-N-(2-(1H-tetrazol-5-yl)phenyl-6-trifluoromethyl-2H-thieno-[3,2-e]-1,2-thiazine-3-carboxamide-1,1-dioxide.
   (R₁ = hydrogen atom, Ar = trifluoromethyl-substituted thiophene ring)
(42) 4-hydroxy-2-methyl-N-(2-(1H-tetrazol-5-yl)phenyl)-6-chloro-2H-thieno-[3,2-e]-1,2-thiazine-3-carbozamide-1,1-dioxide.
   (R₁ = hydrogen atom, Ar = chlorine-substituted thiophene ring)
(43) 4-hydroxy-2-methyl-N-(2-(1H-tetrazol-5-yl)phenyl)-2H-quino[6,7-e]-1,2-thiazine-3-carboxamide-1,1-dioxide.
   (R₁ = hydrogen atom, Ar = quinoline ring)
(44) 4-hydroxy-2-methyl-N-(2-(1H-tetrazol-5-yl)phenyl)-2H-isoquino-[6,7-e)-1,2-thiazine-3-carboxamide-1,1-dioxide.
   (R₁ = hydrogen atom, Ar = isoquinoline ring)
Pharmaceutically acceptable salts of the above compounds (1) to (44) (e.g. alkali metal salts such as sodium salts) are also preferably used in the present invention. The present inventors have found that sodium salts are excellent in solubility and particularly have an excellent therapeutical effect on renal diseases and digestive organ inflammations.

The form of the medicament obtained according to the use of the present invention is selected from forms of oral solid preparations such as powders, grains, granules, tablets, coated tablets, capsules, etc., oral liquid preparations such as syrup, etc., or injections. The preparations can be formed in the presence of a conventional preparation carrier according to conventional methods.

That is, when an oral solid preparation is formed, an excipient is added to the above active component, and optionally, a binder, a disintegrant, a surface smoother, a colorant and a flavor are added. Then, the mixture is formed into powders, grains, granules, tablets, coated tablets, capsules, etc., according to conventional methods.

The excipient is selected from lactose, corn starch, saccharose, glucose, sorbitol, crystalline cellulose, silicon dioxide, etc. The binder is selected from polyvinyl alcohol, polyvinyl ether, ethyl cellulose, methyl cellulose, gum arabic, tragacanth, gelatin, shellac, hydroxypropyl cellulose, hydroxypropyl starch, polyvinyl pyrrolidone, etc. The disintegrant is selected from starch, agar, gelatin powder, crystalline cellulose, calcium carbonate, sodium hydrogen carbonate, calcium citrate, dextrin, pectin, etc. The surface smoother is selected from magnesium stearate, talc, polyethylene glycol, silica, hardened plant oil, etc. The colorant is selected from substances of which the use in medicaments is allowed. The flavor is selected from cocoa powders, menthol, aromatic acid, peppermint oil, refined Borneo camphor, cinnamon powder, etc. In addition, when orally administering solid preparations are formed, the above preparations may be naturally coated with sugar, gelatin, etc.

When the oral liquid preparation is formed, for example, a conventional syrup (solution) is obtained by adding saccharose, sorbitol, etc., as an edulcorant, adding sorbitan fatty acid ester, polysorbate, polyvinyl pyrrolidone, ethylenediamine, glycerin, etc., as a dissolving aid, and optionally adding p-oxybenzoic acid esters, sodium benzoate, benzyl alcohol, sodium dihydroacetate, etc., as a preservative. A syrup suspension is formed by adding gum arabic, tragacanth, sodium carboxymethyl cellulose, methyl cellulose, etc., as a suspension aid in addition to the above preparation materials.

When the injection is formed, the solvent is selected from aqueous solvents such as distilled water for injection, physiological saline solution, Ringer's solution, etc., nonaqueous solvents such as plant oils (sesame oil, peanut oil, corn oil, etc.), fatty acid esters (ethyl oleate, etc.), ethanol, propylene glycol, glycerin, etc., and a mixture of an aqueous solvent with an alcoholic nonaqueous solvent. In addition, a stabilizer, a dissolving aid, a suspension former, an emulsifier, a buffer (pH regulator), an isotonicity former, a pain remover, a preservative, etc., are added.

The stabilizer is selected from antioxidants such as L-ascorbic acid, sodium pyrosulfite, sodium hydrogensulfite, rongalite, etc. The dissolving aid is selected from polyoxyethylene hardened castor oil, ethanol, sodium hydroxide, sodium hydrogen carbonate, ethylenediamine, etc. The suspension former is selected from sodium carboxymethyl cellulose, aluminum monostearate, etc. The emulsifier is selected from polyoxyethylene hardened castor oil. The buffer (pH regulator) is selected from phosphoric acid salts, hydrochloric acid, sodium hydroxide, etc. The isotonicity former is selected from sodium chloride, glucose, glycerin, etc. The pain remover is selected from benzyl alcohol, procaine hydrochloride, dibucaine hydrochloride, lidocaine hydrochloride, gluclose, inositol, etc. The preservative is selected from sodium p-oxybenzoate, benzyl alcohol, chlorobutanol, phenol, cresol, etc.

The dose of the preparation obtained according to the use of the present invention differs depending upon the degrees of diseases, age and health state of a patient, etc., and therefore, cannot be fixed. In general, however, the preparation of the present invention can be administered at such a dose that the amount of the thiazine-1,1-dioxide derivative as an active component is 10 to 1,000 mg/kg/day, whereby the desired effects can be obtained.

The following are Examples of the present invention.

### Example 1

### Preparation of thiazine-1,1-dioxide derivative

6 Parts by weight of 4-hydroxy-2-methyl-3-methoxycarbonyl-2H-1,2-benzothiazine-1,1-dioxide and 3.6 parts by weight of 2-(1H-tetrazol-5-yl)aniline were mixed with each other, and the resultant mixture was condensed by refluxing it in 100 parts by weight of o-xylene under heat for 24 hours. While the resultant reaction mixture was hot, impurities were removed by filtration, and the filtrate was washed with o-xylene. The filtrate was subjected to recrystallization from dioxane-water to give a derivative of the general formula (I) in which R₁ is a hydrogen atom and Ar is a benzene ring, i.e. 4-hydroxy-2-methyl-N-(2-(1H-tetrazol-5-yl)phenyl)2H-1,2-benzothiazine-3-carboxamide-1,1-dioxide (to be referred to as HX-1920 hereinafter).

### Example 2

### Effect on nephritis caused by cisplatin -1

Five female Fischer rats (5 weeks of age) per group were administered hypodermically with 2 mg/kg of cisplatin for 5 days, and at the same time, HX-1920 was administered orally in a dose of 50 mg or 150 mg/kg, or administered intraperitoneally in a dose of 10 or 30 mg/kg. Twenty-four hours after the final administration, the rats were sacrificed, and their blood urea nitrogen (BUN) and creatinine were measured.

As shown in Table 1, in all of the administrations, the blood urea nitrogen tended to show a lower value. In particular, the intraperitoneal administration in a dose of 30 mg/kg showed a significant decrease (P<0.05), and the effect on the alleviation of renal dysfunctions was observed.

**Table 1**

| Drug administration (mg/kg/day) | BUN (mg/dl) | Creatinine (mg/dl) |
|---|---|---|
| Vehicle control group | 149.8±39.7 | 0.67±0.11 |

| HX-1920 administered groups | | |
|---|---|---|
| 10 mg i.p. | 102.8±29.5 | 0.70±0.04 |
| 30 mg i.p. | 69.6±22.2* | 0.65±0.04 |
| 50 mg p.o. | 138.1±47.6 | 0.83±0.31 |
| 150 mg p.o. | 91.1±58.6 | 0.65±0.11 |

| | | |
|---|---|---|
| * shows a significant difference from control value (P<0.05). | | |

### Example 3

### Effect on nephritis caused by cisplatin - 2

Six female Fischer rats (8 weeks of age) per group were administered hypodermically with 1 mg/kg of cisplatin for 8 days, and at the same time, HX-1920 was administered orally in a dose of 10 mg or 100 mg/kg. In both the administrations, the blood urea nitrogen (BUN) and creatinine measured 24 hours after the final administration tended to show lower values. In particular, the group to which 100 mg/kg had been administered showed a significant decrease, and the effect on the alleviation of renal dysfunctions was observed.

**Table 2**

| Drug administration (mg/kg/day) | BUN (mg/dl) | Creatinine (mg/dl) |
|---|---|---|
| Vehicle control group | 61.5±10.4 | 1.28±0.23 |

| HX-1920 administered groups | | |
|---|---|---|
| 10 mg p.o. | 51.1±16.5 | 1.15±0.29 |
| 100 mg p.o. | 38.0±6.4* | 0.96±0.05 |

| | | |
|---|---|---|
| * P<0.05 | | |

### Example 4

### Effect on nephritis caused by cisplatin - 3

Ten male ICR rats (8 weeks of age) per group were administered subcutaneously with 3 mg/kg of cisplatin for 4 days, and at the same, HX-1920 was orally administered in a dose of 100 mg/kg. Sixteen hours after the final administration, 5 rats of each group were measured for blood urea nitrogen (BUN), and the survival ratios of the remaining 5 rats of each group for the subsequent 5 days were examined. As shown in Table 3, due to the administration of HX-1920, BUN showed a low value. Thus, the effect of the administration of HX-1920 on the alleviation of renal dysfunctions was observed. Further, all of the rats of the vehicle control group died, whereas all of the five rats of the HX-1920 administered group survived.

**Table 3**

| Drug administration (mg/kg/day) | Number of rats (n) | BUN (mg/dl) | Survival ratio (%) |
|---|---|---|---|
| Vehicle control group | 5 | 206.4±35.4 | 0 |

| HX-1920 administered groups | | | |
|---|---|---|---|
| 100 p.o. | 5 | 78.3±45.7 | 100 |

### Example 5

### Effect on nephritis caused by cisplatin - 4

Six to seven female F344 rats per group weighing about 150 g, per group, were administered hypodermically in the dorsal portion with 1 mg/kg of cisplatin dissolved in a physiological saline solution once a day over 8 days. Twenty-four hours after the final administration of the cisplatin, the rats were anesthtized with ether, and the blood was collected from each rat, centrifuged and measured for blood urea nitrogen (BUN) and creatinine with an autoanalyzer Olympus AU-550.

HX-1920 which was a substance to be tested was dissolved in a 2.5 % NaHCO₃, and the resultant solution was administered orally in a dose of 10 or 100 mg/kg over 8 days immediately before the administration of the cisplatin.

The results are shown in Table 4. As compared with the group to which no cisplatin was administered, the vehicle control group to which the cisplatin had been administered showed a significant increase in BUN and creatinine. As compared with this increase, the administration of HX-1920 in a dose of 10 mg/kg did not exhibit clear inhibition activity. However, the administration thereof in a dose of 100 mg/kg exhibited inhibition activities of 38 % in BUN and 25 % in creatinine. The increases in BUN and creatinine by the administration of cisplatin are considered to be characteristics in acute renal failure. HX-1920 exhibited the inhibition activity against the increases in BUN and creatinine induced by cisplatin, which shows that HX-1920 has usefulness against renal dysfunctions caused by cisplatin.

**Table 4**

| Drug administration (mg/kg/day) | BUN (mg/dl) | Creatinine (mg/dl) |
|---|---|---|
| Cisplatin non-administered group | 19.9±0.9 | 0.44±0.06 |
| Vehicle control group | 61.5±10.4 | 1.28±0.23 |

| HX-1920 administered groups | | |
|---|---|---|
| 10 mg p.o. | 51.1±16.5 | 1.15±0.29 |
| 100 mg p.o. | 38.0±6.1* | 0.96±0.05 |

| | | |
|---|---|---|
| * P<0.05 | | |

### Example 6

### Effect on inhibition of vomiting caused by cisplatin -1

Five dogs (7 - 12.5 kg) per group were admininistered intravenously with 0.4 mg/kg of cisplatin per day for the initial 3 days and with 0.8 mg/kg of cisplatin per day for the subsequent 1 day. HX-1920 in a dose of 20 mg/kg was encapsulated and administered 30 minutes before the intravenous administration of cisplatin. Separately, Nauzelin (trade name, manufactured by Kyowa Hakko Kogyo Co., Ltd.), which was a domperidone type antiemetic, was administered to another group of five dogs in a dose of 2 mg/kg for 7 days. On the fifth day, the dogs of the vehicle control group and the Nauzelin administered group began to squat down with their abdominal portion on the floor, and then showed vomiting. The vomiting was cured after 24 hours. However, the same symptom was repeatedly manifested when cisplatin was administered. With regard to the HX-1920 administered group, a few dogs showed a slight vomiting symptom as shown in Table 5, however, the remaining dogs showed a normal state. Thus, the effect of HX-1920 on the alleviation of the vomit-inducing activity of the carcinostatic cisplatin was evident.

**Table 5**

| Drug | Animal number | Degree of vomiting | | |
|---|---|---|---|---|
| | | 5th day | 6th day | 7th day |
| Vehicle control group | 1 | III | III | III |
| | 2 | II | II | II |
| | 3 | II | II | II |
| | 4 | III | III | III |
| | 5 | I | II | II |
| HX-1920 administered group 20 mg/kg p.o. | 6 | 0 | I | I |
| | 7 | I | 0 | I |
| | 8 | 0 | 0 | 0 |
| | 9 | 0 | I | I |
| | 10 | 0 | 0 | 0 |
| Nauzelin administered group 2 mg/kg p.o. | 11 | III | III | III |
| | 12 | II | II | III |
| | 13 | II | II | III |
| | 14 | II | II | II |
| | 15 | III | III | III |
| Remarks III: Extreme II: Moderate I: Slight 0: No vomiting | | | | |

### Example 7

### Effect on inhibition of vomiting caused by cisplatin -2

Female and male mongrel dogs weiging about 10 kg were administered intravenously with 0.4 mg/kg/day of cisplatin dissolved in a physiological saline solution for the initial 3 days and with 0.8 mg/kg/day of cisplatin for the subsequent 4 days to examine the effects of substances to be tested on the basis of a manifestation ratio, as an index, of vomiting induced by the cisplatin.

HX-1920, which was a substance to be tested, was dissolved in a 10 % Nikkol solution (Nikkol: trade name, dissolving aid manufactured by Nikko Chemical Co., Ltd.) containing 1 % NaHCO₃, and put into gelatin soft capsules, and the capsules were administered orally 30 minutes before the administration of cisplatin.

In addition, as a known domperidone type antiemetic, a 1 % Nauzelin powder was put into gelatin soft capsules, and administered orally 30 minutes before the administration of cisplatin.

As shown in the vehicle group, the vomiting was clearly observed on and after the fourth day after the administration of cisplatin, and on the 7th day, 17 out of 18 dogs showed vomiting (manifestation ratio 94.4 %). The Nauzelin administered group showed that Nauzelin was not effective against vomiting. However, in the HX-1920 administered group, the manifestation of vomiting was clearly inhibited as compared with the vehicle control group. These data show that HX-1920 is a useful compound for the inhibition of vomiting which is a side effect of the carcinostatic cisplatin.

**Table 6**

| Drug | n | Vomiting manifestation ratio (%) | | | |
|---|---|---|---|---|---|
| | | 4th day | 5th day | 6th day | 7th day |
| Vehicle control group | 18 | 44.4 | 66.7 | 77.8 | 94.4 |
| HX-1920 administered group 20 mg/kg | 17 | 29.4 | 23.5 | 29.4 | 52.9 |
| Nauzelin administered group 2 mg/kg | 8 | 50.0 | 75.0 | 100 | 100 |

### Example 8

### Effect on inhibition of stomach contraction caused by cisplatin

Cisplatin in a dose of 2 mg/kg was administered subcutaneously into female Fischer rats (5 weeks of age) once a day for 4 days, and at the same time, HX-1920 was administered intraperitoneally or orally.

The dose of HX-1920 for the intraperitoneal administration on the basis of the rat body weight was 10 mg/kg or 30 mg/kg, and that for the oral administration was 50 mg/kg or 150 mg/kg. For the cisplatin non-administered group and the cisplatin administered vehicle control group, 1 ml/day of a 1 % gum arabic solution was administered per rat.

As a result, the groups to which HX-1920 had been administered showed that the diameter of the center of the stomach thereof measured a nearly normal value, and showed a clear significant difference from the cisplatin administered vehicle control group.

**Table 7**

| Dose | Diameter of center of stomach (mm) |
|---|---|
| Cisplatin non-administered group | 1.16±0.04 |
| Vehicle control group | 0.84±0.05 |

| HX-1920 administered groups | |
|---|---|
| 10 mg i.p. | 0.97±0.07 |
| 30 mg i.p. | 1.18±0.07 |
| 30 mg p.o. | 1.13±0.11 |
| 150 mg p.o. | 1.14±0.07 |

As detailed above, according to the present invention, there is provided a therapeutic preparation for renal diseases, which has excellent therapeutical effect on renal diseases.

Further, according to the present invention, there is provided a therapeutic preparation for a digestive organ, which can alleviate vomiting, nausea, anorexia, stomach contraction, etc., when a carcinostatic or other medicament is administered.

## Claims

1. Use of a thiazine-1,1-dioxide derivative of the general formula (I), 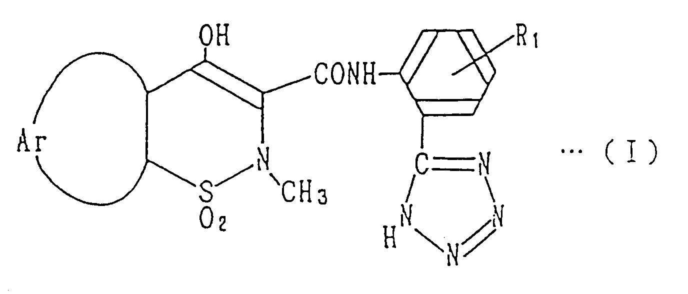 (wherein R₁ represents an atom or a substituent selected from the class consisting of a hydrogen atom, an alkyl group, an alkoxyl group, a halogen atom, a halogen-containing alkyl group, a hydroxyl group, a carboxyl group, alkylcarbonyl group and an ester group, Ar is (a) a benzene or naphthalene ring fused with the thiazine ring in the general formula (I) or (b) a hetero atom-containing aromatic ring selected from thiophene, furan, pyridine, quinoline, isoquinoline, and imidazole rings fused with the thiazine ring in the general formula (I)), or a pharmaceutically acceptable salt thereof for the manufacture of a medicament, for the treatment of renal diseases or for the treatment of disorders of digestive organs, e.g. vomiting, nausea, anorexia, stomach contractions.

2. Use according to claim 1, wherein the alkyl group included in R₁ is a linear or branched alkyl group having 1 to 6 carbon atoms.

3. Use according to claim 1, wherein the alkoxyl group included in R₁ is an alkoxyl group having a linear or branched alkyl group having 1 to 6 carbon atoms.

4. Use according to claim 1, wherein the halogen atom included in R₁ is chlorine, fluorine, bromine or iodine.

5. Use according to claim 1, wherein the halogen-containing alkyl group included in R₁ is a linear or branched alkyl group having 1 to 6 carbon atoms and being substituted with at least one halogen atom.

6. Use according to claim 1, wherein the alkylcarbonyl group included in R₁ is an alkylcarbonyl group having a linear or branched alkyl group having 1 to 6 carbon atoms.

7. Use according to claim 1, wherein the ester group included in R₁ is an ester group of the formula of COOR₂ (in which R₂ is linear or branched alkyl group having 1 to 6 carbon atoms).

## Patentansprüche

1. Verwendung eines Thiazin-1,1-dioxid Derivats der allgemeinen Formel (I), 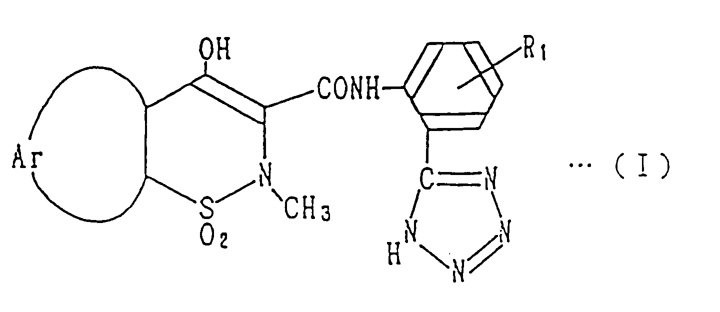 (wobei R 1 ein Atom oder ein Substituent, ausgewählt aus der Klasse bestehend aus einem Hydrogen-Atom, einer Alkyl-Gruppe, einer Alkoxyl-Gruppe, einem Halogen-Atom, einer Halogen-enthaltenden Alkyl-Gruppe, einer Hydroxyl-Gruppe, einer Carboxyl-Gruppe, Alkylcarbonyl-Gruppe und einer Ester-Gruppe darstellt, Ar (a) ein Benzol-oder Naphthalinring verschmolzen mit dem Thiazinring in der allgemeinen Formel (I) ist, oder (b) ein ein hetero-Atom-enthaltender aromatischer Ring, ausgewählt aus Thiophen, Furan, Pyridin, Chinolin, Isochinolin und Imidozolinringen verschmolzen mit dem Thiazinring in der allgemeinen Formel(I)), oder ein pharmazeutisch annehmbares Salz davon, zur Herstellung eines Medikaments für die Behandlung von Nierenkrankheiten oder für die Behandlung von Störungen der Verdauungsorgane e.g. Erbrechen, Übelkeit, Anorexie, Magenkontraktionen.

2. Die Verwendung entsprechend Anspruch 1, wobei die in R1 enthaltene Alkylgruppe eine lineare oder verzweigte Alkylgruppe mit 1 bis 6 Kohlenstoffatomen ist.

3. Die Verwendung entsprechend Anspruch 1, wobei die in R1 enthaltene Alkylgruppe eine Alkoxylgruppe mit einer linearen oder verzweigten Alkoxylgruppe mit 1 bis 6 Kohlenstoffatomen ist.

4. Die Verwendung entsprechend Anspruch 1, wobei das in R1 enthaltene Halogen-Atom Chlor, Fluor, Brom oder Jod ist.

5. Die Verwendung entsprechend Anspruch 1, wobei die in R1 enthaltene Halogen-enthaltende Alkylgruppe eine lineare oder verzweigte Alkylgruppe mit 1 bis 6 Kohlenstoffatomen und substituiert durch zumindest ein Halogenatom ist.

6. Die Verwendung entsprechend Anspruch 1, wobei die in R1 enthaltene Alkylcarbonylgruppe eine Alkylcarbonylgruppe mit einer linearen oder verzweigten Alkylgruppe mit 1 bis 6 Kohlenstoffatomen ist.

7. Die Verwendung entsprechend Anspruch 1, wobei die in R1 enthaltene Estergruppe eine Estergruppe der Formel COOR2 ist,(in welcher R2 eine lineare oder verzweigte Alkylgruppe mit 1 bis 6 Kohlenstoffatomen ist).

## Revendications

1. Application d'un dérivé de thiazine-1,1-dioxyde de formule générale (I), 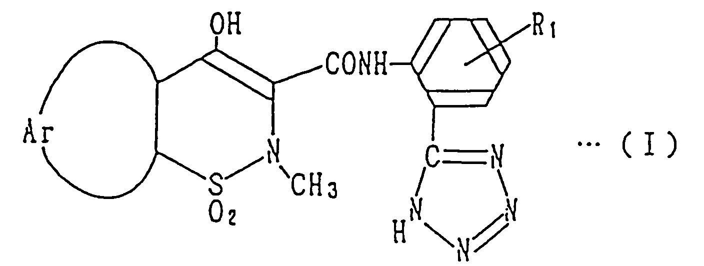 (dans laquelle R₁ représente un atome ou un substituant choisi dans la classe constituée par un atome d'hydrogène, un groupe alkyle, un groupe alcoxyle, un atome d'halogène, un groupe alkyle contenant un halogène, un groupe hydroxyle, un groupe carboxyle, un groupe alkylcarbonyle et un groupe ester ; Ar est (a) un noyau benzène ou naphtalène condensé avec le noyau thiazine dans la formule générale (I) ou (b) un noyau aromatique contenant un hétéroatome choisi parmi les noyaux thiophène, le furanne, la pyridine, la quinoléine, l'isoquinoléine, et imidazole condensés avec le noyau thiazine dans la formule générale (I)), ou d'un de ses sels pharmaceutiquement acceptables pour la préparation d'un médicament visant à traiter les maladies rénales ou visant à traiter les maladies des organes digestifs, par exemple les vomissements, la nausée, l'anorexie et les contractions de l'estomac.

2. Application selon la revendication 1, dans laquelle le groupe alkyle inclus dans R₁ est un groupe alkyle linéaire ou ramifié ayant de 1 à 6 atomes de carbone.

3. Application selon la revendication 1, dans laquelle le groupe alcoxyle inclus dans R₁ est un groupe alcoxyle ayant un groupe alkyle linéaire ou ramifié ayant de 1 à 6 atomes de carbone.

4. Application selon la revendication 1, dans laquelle l'atome d'halogène inclus dans R₁ est le chlore, le fluor, le brome ou l'iode.

5. Application selon la revendication 1, dans laquelle le groupe alkyle contenant un halogène inclus dans R₁ est un groupe alkyle linéaire ou ramifié ayant de 1 à 6 atomes de carbone et substitué par au moins un atome d'halogène.

6. Application selon la revendication 1, dans laquelle le groupe alkylcarbonyle inclus dans R₁ est un groupe alkylcarbonyle ayant un groupe alkyle linéaire ou ramifié comprenant de 1 à 6 atomes de carbone.

7. Application selon la revendication 1, dans laquelle le groupe ester inclus dans R₁ est un groupe ester de formule COOR₂ (dans laquelle R₂ est un groupe alkyle linéaire ou ramifié ayant de 1 à 6 atomes de carbone).
